# EUROPEAN PATENT SPECIFICATION

(11) **EP 0 690 722 B1**
(45) Date of publication and mention of the grant of the patent: **30.06.2004**
(21) Application number: 94911691.7
(22) Date of filing: 18.03.1994
(51) Int. Cl.: A61K 39/00, A61K 9/14, A61K 33/38, A61K 33/24, A61K 45/00

(54) **COMPOSITION AND METHOD FOR REDUCING TOXICITY OF BIOLOGICALLY-ACTIVE FACTORS**
ZUSAMMENSETZUNG UND METHODE ZUR REDUKTION DER TOXIZITÄT VON BIOLOGISCH-AKTIVEN FAKTOREN
COMPOSITION ET METHODE D'ABAISSEMENT DE LA TOXICITE DE FACTEURS BIOLOGIQUEMENT ACTIFS

(30) Priority: 18.03.1993 US 33385
(43) Date of publication of application: 10.01.1996
(73) Proprietor: CYTIMMUNE SCIENCES, INC., College Park, MD 20740 (US)
(72) Inventor: TAMARKIN, Lawrence, Rockville, MD 20854 (US); PACIOTTI, Giulio Franco, Baltimore, MD 21227 (US)
(74) Representative: Sternagel, Fleischer, Godemeyer & Partner Patentanwälte
(86) International application number: PCT/US1994/003177
(87) International publication number: WO 1994/021288

(56) References cited:
- EP-A- 0 269 408
- WO-A-91/02078
- FR-A- 2 334 366
- US-A- 3 269 912
- US-A- 5 112 606
- SCIENCE, vol. 260, 14 May 1993, pages 937-944, XP002030454 LANZAVECCHIA: "IDENTIFYING STRATEGIES FOR IMMUNE INTERVENTION"
- Journal of Experimental Medicine, Volume 17, issued September 1989, FRAKER et al., "Passive Immunization Against Tumor Necrosis Factor Partially Abrogates Interleukin 2 Toxicity", pages 1015-1020, see pages 1015-1016 and 1019.
- Journal of Histochemistry and Cytochemistry, Volume 37, No. 2, issued February 1989, COULOMBE et al., "Cytochemical Demonstration of Increased Phospholipid Content in Cell Membranes in Chlorphentermine-Induced Phospholipidosis", pages 139-147, see pages 139 and 140.
- Brain Research, Volume 540, issued 01 February 1991, HASHIMOTO et al., "Action Site of Circulating Interleukin-1 on the Rabbit Brain", pages 217-223, see pages 217-218.
- Lymphokine Research, Volume 9, No. 1, issued 1990, OHMANN et al., "Expression of Tumor Necrosis Factor-Alpha Receptors on Bovine Macrophages, Lymphocytes and Polymorphonuclear Leukocytes, Internalization of Receptor-Bound Ligand, and Some Functional Effects", pages 43-58, see page 44.

## Description

### Technical Field

The present invention relates to compositions and methods for reducing the toxicity of biologically-active factors, such as cytokines and growth factors. In addition, the present invention comprises vaccines which are effective in immunizing a human or animal against a biologically-active factor while reducing or eliminating the toxicity of the factor. More particularly, the present invention relates to compositions comprising a colloidal metal in combination with a biologically-active factor which renders the biologically-active factor non-toxic.

### Background of the Invention

Various biologically active compounds have been isolated from humans or animals which have been reported to have therapeutic efficacy. These compounds include cytokines and growth factors. However, it has been found that when these various factors are isolated and purified and then injected into a human or animal, they often cause severe side effects and exhibit unwanted toxicity. Because of this toxicity, it has been difficult to use the compounds therapeutically. In addition, it has been difficult to use the active compounds as antigens to produce antibodies against the molecules.

Fraker, D. L., Langstein, H. N. and Norton, J. A. describe in Journ. of Exp. Med., Vol. 71, 1989, 1015-1020 the toxicity of Interleucin-2 (IL-2) when used for human malignancies. Toxicity is based on the upregulation of TNF, which has an effect as mediator of toxicity in endotoxemia and Gram-negative sepsis. By injecting anti-TNF antibodies concurrently with the IL-2 treatment the toxic effect of IL-2 can be decreased.

Aluminum compounds have been used to form water-insoluble antigenic substances. For example, U.S. Patent No. 3,577,523, issued to *Stolar, et al*., discloses the combination of aluminum tannate with antigenic extracts to form water-insoluble slow release antigenic substances. More generally, the *Stolar*, *et al*. patent discloses the use of antigenic depot agents incorporating water-insoluble antigenic substances that slowly release active agents that are absorbed without adverse systemic reactions or other adverse side effects.

Metals have also been used in capsular polysaccharide metal complex vaccines. For example, in U.S. Patent No. 4.740,589, issued to *Moreno*, a bacterial capsular polysaccharide constituent was complexed with a metal, preferably aluminum or ruthenium, for the prophylaxis and treatment of bacterial diseases. This patent aiso discloses the formulation of a three component complex which contains a polysaccharide, a metal, and a third constituent of bacterial outer-membrane protein. The '589 patent discloses that the complex contains a weight percentage of lipopolysaccharide "insufficient to produce significant toxic effects", the weight percentage being generally 1% or less. Finally, the disclosure in the '589 patent application is limited to the use of the complexes for prophylaxis and treatment of bacterial diseases.

U.S. Patent No. 3,269,912, issued to *Grase*, discloses a depot vaccine comprising a finely divided aluminum oxide, either aluminum oxide or aluminum oxide aerosol having had absorbed thereon at least one antigen derived from a virus, bacteria, or ectotoxoid, dispersed in an aqueous medium. The '912 patent also discloses that the vaccine forms a colloidal dispersion of the individual spherical crystals of aluminum oxide in the solution.

Selected metals have also been used as components of stable adjuvant emulsion compositions. It is known in the art that aluminum, as the monostearate, or in the form of hydrated salts of fatty acids, are emulsifying agents, or stabilizers of the emulsion in the vaccine composition.

However, substantial need exists for a therapeutically effective composition with reduced toxicity, that may be used in therapies for a wide range of immune diseases, cancers, viral diseases and bacterial diseases. In addition, there is a need for a composition that can reduce the toxicity of normally toxic biologically-active compositions so that the compounds can be used as vaccines in the human or animal.

### Summary of the Invention

The present invention satisfies the above-described needs by providing effective compositions containing normally toxic compounds that, when in combination with a colloidal metal, result in significantly reduced toxic side effects. Generally, the composition of the present invention comprises an admixture of a colloidal metal, such as gold chloride (HAuCl₄) in combination with a substance which normally is toxic to a human or animal capable of producing an immune response, wherein the composition when administered to a human or animal is less or non-toxic.

The present application provides a composition for therapeutic use comprising an admixture of a colloidal metal and an immunologically toxic biologically-active factor, selected from the group consisting of cytokines, growth factors, heat shock proteins, carbohydrate moieties of blood groups, Rh factors, glycoproteins from infectious organisms, inflammatory proteins, lipid A, endotoxins and immune regulatory proteins, wherein if the factor is selected from the group consisting of endotoxins, and wherein such endotoxin is lipopolysaccharide, the colloidal metal is not aluminium oxide.

The above composition is for use in administration to a human or animal an effective amount of a composition comprising a colloidal metal, such as HAuCl₄, in combination with a substance which normally is toxic to a human or animal capable of producing an immune response, wherein the composition, when administered to a human or animal, is less toxic or non-toxic.

The use of colloidal metals in combination with normally toxic biologically active compounds in the manufacture of a medicament may be used in cancer and immune disease therapies. Current therapies which consist of administering factors such as interleukins to a human or animal are marginally effective but produce significant, toxic side effects. Also, the toxic side effects limit the amount of biologically-active factors that may be administered, and therefore limits the efficacy of the therapy. Additionally, some otherwise therapeutic compounds are not used at all due to their toxicity. The combination of a colloidal metal with such biologically-active factors reduces toxicity while maintaining the therapeutic effectiveness.

Accordingly, it is an object of the present invention to provide a composition that is capable of reducing the toxicity of biologically-active factors.

A further object of the present invention is to provide a composition containing higher concentrations of biologically-active factors than are currently utilized due to the toxicity of the substances.

Another object of the present invention is to provide a composition to be used in cancer therapies which results in reduced side effects from toxicity.

Yet another object of the present invention is to provide a composition for immune disease therapy which results in reduced side effects from toxicity.

Another object of the present invention is to provide a composition or immune disease therapy which results in reduced toxic side effects and maintains its beneficial effects.

Yet another object of the present invention is to provide a composition for vaccinating a human or animal against a normally toxic biologically-active factor..

These and other objects, features and advantages of the present invention will become apparent after a review of the following detailed description of the preferred embodiment.

### Brief Description Of The Drawings

Figure 1 illustrates the serum antibody titers in mice immunized with murine IL-6 combined with colloidal gold.
Figure 2 illustrates the retention of biologic activity of IL-1 after treatment with gold.
Figure 3 illustrates the efficiency with which gold binds IL-6.

### Detailed Description

The terms "toxic reaction," and "toxicity," as used herein, include, but are not limited to, the following responses of an animal or human: fever; edema, including cerebral edema; psychosis; autoimmune diseases; hemorrhage; shock, including hemorrhagic shock; sepsis; cachexia; or death. The term "colloidal metal", as used herein, includes any water-insoluble metal particle or metallic compound dispersed in liquid water (a hydrosol). The term "biologically-active factors" includes, but is not limited to, Interleukin-2 ("IL-2"), lipid A, phospholipase A2, endotoxins, staphylococcal enterotoxin B and other toxins, Type I Interferon, Type II Interferon, Tumor Necrosis Factor, IL-1, IL-6, IL-8, IL-4, Transforming Growth Factor-B, Lymphotoxin, IL-5, Migration Inhibition Factor, IL-3, Granulocyte-Macrophage Colony-Stimulating Factor ("CSF"), Monocyte-Macrophage CSF, Granulocyte CSF, IL-7, IL-10, IL-11, IL-12, IL-13, vascular epithelial growth factor ("VEGF"), Angiogenin, transforming growth factor ("TGFα"), heat shock proteins, carbohydrate moieties of blood groups, Rh factors, fibroblast growth factor, and other inflammatory and immune regulatory proteins.

The present invention comprises a composition for administering normally toxic biologically-active factors to a human or animal. Generally, the composition according to the present invention comprises an admixture of a colloidal metal in combination with a substance which normally is toxic to a human or animal capable of producing an immune response, wherein the composition, when administered to a human or animal, is less or non-toxic to the human or animal. The composition optionally includes a pharmaceutically-acceptable carrier, such as an aqueous solution, or excipients, buffers, antigen stabilizers, or sterilized carriers. Also, oils, such as paraffin oil, may optionally be included in the composition.

The composition of the present invention can be used to vaccinate a human or animal against biologically-active factors which are normally toxic when injected. In addition, the present invention can be used to treat certain diseases with cytokines or growth factors. By admixing the biologically-active factors with the colloidal metal before administering them to the human or animal, the toxicity of the biologically-active factor is reduced or eliminated thereby allowing the factor to exert its therapeutic effect.

Current therapies which comprise administering biologically-active factors to a human or animal are somewhat effective yet produce significant toxic side effects. Further, the toxic side effects limit the amount of antigen that may be administered, and therefore limit the efficacy of the therapy. Additionally, the toxicity of some biologically-active factors precludes their use in such therapies. The combination of a colloidal metal with such biologically-active factors reduces toxicity while maintaining or increasing the therapeutic results thereby improving the efficacy as higher concentrations of biologically-active factors may be administered, or by allowing the use of combinations of biologically-active factors. The use of colloidal metals in combination with biologically-active factors therefore allows the use of higher concentrations of biologically-active factors or formerly unusable toxic substances, to be administered to humans or animals.

The term "biologically-active factors" includes, but is not limited to, Interleukin-2 ("IL-2"), lipid A, phospholipase A2, endotoxins, staphylococcal enterotoxin B and other toxins, Type I Interferon, Type II Interferon, Tumor Necrosis Factor, IL-1, IL-6, IL-8, IL-4, Transforming Growth Factor-B, Lymphotoxin, IL-5, Migration Inhibition Factor, IL-3, Granulocyte-Macrophage Colony-Stimulating Factor ("CSF"), Monocyte-Macrophage CSF, Granulocyte CSF, IL-7, IL-10, IL-11, IL-12, IL-13, VEGF, Angiogenin, TGFα, heat shock proteins, carbohydrate moieties of blood groups, Rh factors, fibroblast growth factor, and other inflammatory and immune regulatory proteins.

The colloidal metal may for example be selected from the metals in groups IIA, 1B, IIB and IIIB of the periodic table, as well as the transition metals, especially those of group VIII. Preferred metals include gold, silver, aluminum, ruthenium, zinc, iron, nickel and calcium. Other suitable metals may also include the following in all of their various oxidation states: lithium, sodium, magnesium, potassium, scandium, titanium, vanadium, chromium, manganese, cobalt, copper, gallium, strontium, niobium, molybdenum, palladium, indium, tin, tungsten, rhenium, platinum, and gadolinium. The metals are preferably provided in ionic form, (preferably derived from an appropriate metal compound) for example the Al³⁺, Ru³⁺, Zn²⁺, Fe³⁺, Ni²⁺ and Ca²⁺ ions. A preferred metal is gold, particularly in the form of Au³⁺. An especially preferred form of colloidal gold is HAuCl₄ (E-Y Laboratories, Inc., San Mateo, California). Another preferred metal is silver, particularly in a sodium borate buffer, having the concentration of between approximately 0.1% and 0.001%, and most preferably as approximately a 0.01% solution. The color of such a colloidal silver solution is yellow and the colloidal particles range from 1 to 40 nanometers. Such metal ions may be present in the complex alone or with other inorganic ions.

The amount of colloidal metal that is used in the present invention is between approximately 0.001 mg/ml and 1.0 mg/ml with the more preferred amount of colloidal metal being between approximately 0.01 mg/ml and 0.1 mg/ml. The amount of the composition according to the present invention to be administered to humans or animals varies according to the disease to be treated, the biologically-active factor or factors used in the therapy, the species involved, and the physical state of the individual to be treated.

To prepare a vaccine against biologically-active factors, the preparation of the selected biologically-active factor is admixed with the colloidal metal in a salt-free medium, preferably deionized water. The salt-free medium may optionally be buffered with, for example, Tris buffer. In one embodiment of the invention, the colloidal metal solution is diluted 1:1 with the solution of biologically-active factors.

The medium should preferably not contain sodium ions. A colloidal gold solution has a light pink color, this color should not change when adding the solution containing the biologically-active factors. If the colloidal gold solution turns from pink to purple, this indicates that the gold has precipitated and cannot be reconstituted for effective immunization. The shelf-life of an admixture of colloidal gold and biologically active factor(s) is approximately 24 hours.

The admixture of biologically-active factors and colloidal metal is then injected into an appropriate animal. For example, rabbits weighing between approximately two to five kilograms suffered no noticeable side-effects after they were administered, every two weeks, a composition comprising colloidal gold and 1 mg of cytokine, either IL-1 or IL-2. Because the biologically-active factor is not toxic when administered according to the present invention, the optimal quantity of antigen can be administered to the animal. The compositions according to the present invention may be administered in a single dose or they may be administered in multiple doses, spaced over a suitable time scale to fully utilize the secondary immunization response. For example, antibody titers have been maintained by administering boosters once a month.

The vaccine may further comprise a pharmaceutically acceptable adjuvant, including, but not limited to Freund's complete adjuvant, Freund's incomplete adjuvant, lipopolysaccharide, monophosphoryl lipid A, muramyl dipeptide, liposomes containing lipid A, alum, muramyl tripeptide-phosphatidylethanoloamine, keyhole and limpet hemocyanin. A preferred adjuvant is Freund's incomplete adjuvant, which preferably is diluted 1:1 with the mixture of colloidal metal and biologically-active factor.

The composition is for use in administration to a human or animal an effective amount of the composition comprising a colloidal metal admixed with a biologically-active factor or factors, wherein the composition when administered to a human or animal, is less or non-toxic. The composition according to the present invention can be administered as a vaccine against a normally toxic substance or can be a therapeutic agent wherein the toxicity of the normally toxic agent is reduced thereby allowing the administration of higher quantities of the agent over longer periods of time.

In practicing this invention, the process by which the composition is administered is not considered critical. The routes that the composition may be administered according to this invention include, but are not limited to, subcutaneous, intramuscular, intraperitoneal, oral, and intravenous routes. A preferred route of administration is intravenous. Another preferred route of administration is intramuscular.

It is known that Interleukin-2 (IL-2) displays significant therapeutic results in the treatment of renal cancer. However, the toxic side effects result in the death of a significant number of the patients. In contrast, if IL-2 is mixed with colloidal gold, little or no toxicity is observed and a strong immune response occurs. The doses previously used for IL-2 therapy have been on the order of 21x10⁶ units of IL-2 per 70 kg man per day (7x10⁶ units of IL-2 per 70 kg man TID). One unit equals approximately 50 picograms, 2 units equals approximately 0.1 nanograms, so 20x10⁶ units equals 1 milligram. In one embodiment of this invention, the amount of IL-2 that has been given to rabbits is approximately 1 mg per 3 kg rabbit. In effect, the studies of the effects of the administration of biologically-active factors described herein have included doses of more than 20 times higher than that previously given to humans.

In another embodiment, where IL-2 (1 mg per 3 kg animal) was administered to 3 rabbits every third day for a two-week period, all the animals appeared to be clinically sick, and two of the animals died from the apparent toxic effects of the IL-2. When the same dose of IL-2 was combined with colloidal gold and then administered to three rabbits for the same two-week period, no toxicity was observed and a significant antibody response resulted in all three animals. A "positive antibody response" as used herein is defined as a three to fourfold increase in specific antibody reactivity, as determined by direct ELISA, comparing the post-immunization bleed with the pre-immunization bleed. A direct ELISA is done by binding IL-2 onto a microtiter plate, and determining the quantity of IgG bound to the IL-2 on the plate, by goat anti-rabbit IgG conjugated to alkaline phosphatase. Therefore, it is thought that the biological effects of the IL-2 remain. As the toxicity effects have been minimized. larger concentrations of IL-2 may be administered if necessary where a larger, more effective immune response is required.

This invention is further illustrated by the following examples, which are not to be construed in any way as imposing limitations upon the scope thereof. On the contrary, it is to be clearly understood that resort may be had to various other embodiments, modifications, and equivalents thereof which, after reading the description herein, may suggest themselves to those skilled in the art without departing from the scope of the appended claims.

### Example I

This example demonstrates that colloidal gold neutralizes otherwise toxic substances and allows for an antibody response. When IL-2 (1 mg per 3 kg animal) is administered to three rabbits every third day for a two-week period, all the animals appear to be clinically sick, and two of the animals died from the apparent toxic effects of the IL-2. When the same dose of IL-2 is combined with colloidal gold and then administered to three rabbits for the same two-week period, no toxicity is observed and a significant antibody response results in all three animals. A positive antibody response is defined as a three to fourfold increase in specific antibody reactivity, as determined by direct ELISA, comparing the post-immunization bleed with the pre-immunization bleed. A direct ELISA is done by binding IL-2 onto a microtiter plate, and determining the quantity of IgG bound to the IL-2 on the plate, by goat anti-rabbit IgG conjugated to alkaline phosphatase.

### Example II

This example further demonstrates that colloidal gold neutralizes otherwise toxic substances and allows for an antibody response. Endotoxin or lipid A (25, 50, and 100 µg per 35 mg mouse) are administered by subcutaneous injection every fourth day over a two-week period. For ten mice, endotoxin is given "neat" and for the remaining ten, the endotoxin is mixed 1:1 with colloidal gold. The injection volume is made up by adding potassium carbonate/sodium citrate buffer, pH 6.5 at a 1:1 dilution. The same protocol is also used where lipid A is the test drug.

The animals are checked at 15, 30, and 60 minutes following each injection, and then hourly and daily. The surviving animals are tested for a specific antibody response to the toxic substance they were injected with, either endotoxin or lipid A. Most of the animals injected with endotoxin or lipid A combined with colloidal gold survived, while those injected with the neat endotoxin or lipid A died during the two-week test period. In addition, those animals that did survive did have an antibody response to the specific toxin as determined by direct ELISA.

### Example III

This example illustrates the effect of colloidal gold on cytokine activity *in vivo.* A group of mice are given IL-2 at a dose close to that given to cancer patients undergoing immunotherapy. In previous experiments, 18 µg of IL-2 tablets given to nude mice reduced implant tumor size, but killed the animals within two weeks. See Paciotti, G.F., and Tamarkin, L., Interleukin 2 Differentially Effects the Proliferation of a Hormone-Dependent and a Hormone-Independent Human Breast Cancer Cell Line *In Vitro* and *In Vivo, Anti-Cancer Research,* 8: 1233-1240 (1988), which is hereby incorporated by reference.

The efficacy of gold in a murine model system is tested in the following procedure: a group of mice are treated with IL-2 alone, IL-2 mixed with colloidal gold, colloidal gold alone, or saline solutions delivered through an osmotic minipump. The mice are treated for seven days, after which they are sacrificed and their lymphocytes harvested. The cells are stained for T-cell or B-cell markers using specific murine monoclonal antibodies for flow cytometric analysis. Activated T- and B-cells are determined by assessing T-cell numbers, helper T-cell to suppresser T-cell ratios, activated cellular IL-2 receptor, B-cell numbers, and natural killer cell ("NK") numbers.

The few animals that survived being treated with IL-2 alone showed an increase in the T-cell number and activity (as determined by IL-2 receptors). Virtually all the animals survived IL-2 treatment in combination with colloidal gold, and these animals showed an increase in both B-cell function (as determined by activated B-cells and total IgG, measured by direct ELISA) an increase in T-cell function (as determined by T-cell number, and activity, using IL-2 receptor numbers as an index of activity), and an increase in NK activity.

### Example IV

The following biological experiment shows that colloidal gold reduces the toxicity of lipopolysaccharide (LPS). LPS is the lipid/sugar moiety of bacterial cell walls. When injected into an animal, this molecule mimics many of the clinical responses of septic shock. Thus, mice were injected with varying amounts of LPS in the presence or absence of colloidal gold. Specifically Balb/c mice were injected with either 100 or 400 µg of LPS (strain W.E. coli 055:B5; 10 mg/ml in water; Difco Labs) with or without colloidal gold. The pH of the 15 nm colloidal gold mixture (E.Y. Labs) was adjusted to approximately 10, - while the pH of the LPS was adjusted to 8 with 0.1 N NaOH. Subsequently, appropriate volumes (i.e., 10 µl for the 100 µg dose and 40 µl for the 400 µg dose) was then added to 500 µl of colloidal gold. The mixture was allowed to stand for 30 minutes and subsequently injected (i.p.) into the mice.

Within 12 hours after the injections, all mice exhibited clinical signs of depression and anergasia. Within 24 hours after the injection control mice in the 400 µg dose began to die. By 72 hours all of the control mice in the 400 µg dose died while 75% of the gold treated mice were alive and began showing signs of clinical improvement (i.e., movement). Furthermore, although subjective, the mice in the 100 µg dose which were treated with gold were more active throughout the 36 hours of observation.

### Example V

The following experiment describes the use of colloidal gold as a putative adjuvant for generating mouse antibodies against murine IL-6. This experiment was performed with two goals in mind: 1) To determine if colloidal gold could be used as an adjuvant in generating an immune response to "self-antigens" (i.e., generating an immune response to a mouse protein using a mouse model); Secondly, since IL-6 is one of the cytokines thought to be involved in cancer cachexia, metastasis and sepsis, then the ability to generate antibodies in an autologous system may prove advantageous in generating a vaccine to the IL-6 and similar endogenous compounds.

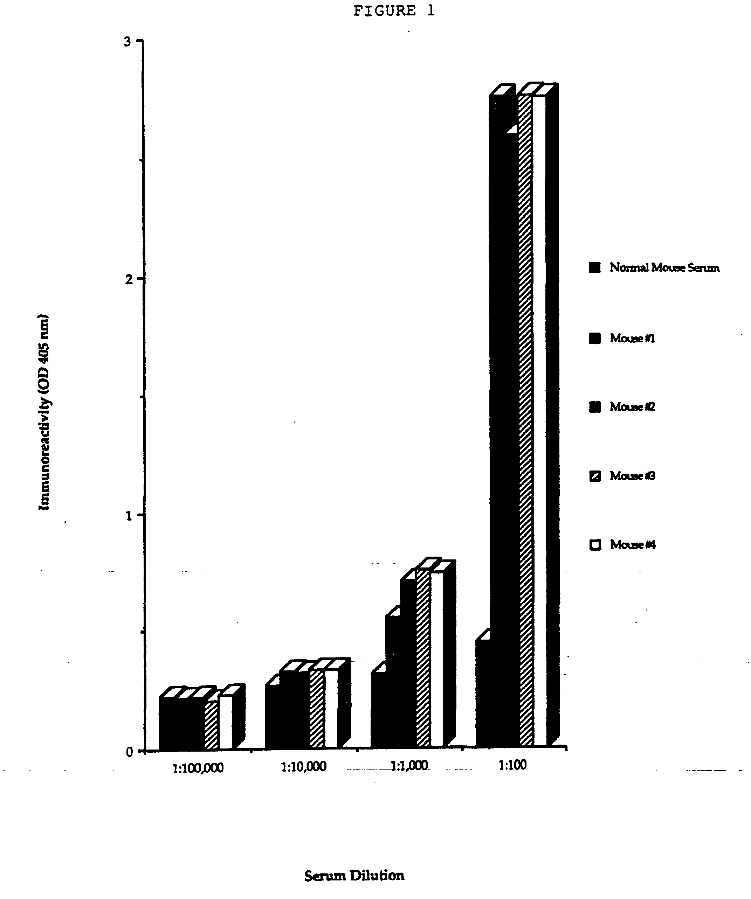

Briefly described, the experiment is as follows. Several mice were immunized with colloidal gold/murine IL-6 mixture as described above. Approximately 3 weeks later, the mice were sacrificed and trunk blood was collected and analyzed for the presence of antibodies to murine IL-6 by a direct ELISA, as described above. The results from the direct ELISA, the determination of the serum antibody titers in mice immunized with murine IL-6 combined with colloidal gold, are illustrated in Figure 1. Figure 1 demonstrates that the mice had generated an antibody response to murine IL-6 thus indicating that the gold may be useful in generating antibodies to endogenous (i.e., self) toxins as well as cytokines thought to be involved in sepsis, cancer cachexia and metastasis.

### Example VI

The following experiment shows that cytokines mixed with colloidal gold retain their biological activity. The model used for these experiments is one which is well known in the art. See Paciotti, G.F., and L. Tamarkin, *Interleukin 1 directly regulates hormone-dependent human breast cancer cell proliferation in vitro,* Mol Endocrinol., 2: 459-464, 1988; and Paciotti, G.F., and L. Tamarkin, *Interleukin-1 differentially synchronizes estrogen-dependent and estrogen-independent human breast cancer cells in the G*_{*0*}/*G*_{*1*}*-phase of the cell cycle,* AntiCancer Research, 11: 25-32, 1991. The model is based on the ability of the cytokine, IL-1, to directly inhibit the growth of estrogen-responsive human breast cancer cells, MCF-7. Briefly described, IL-1 alone inhibits the growth of these cells through a well-characterized IL-1 receptor on the surface of these breast cancer cells.

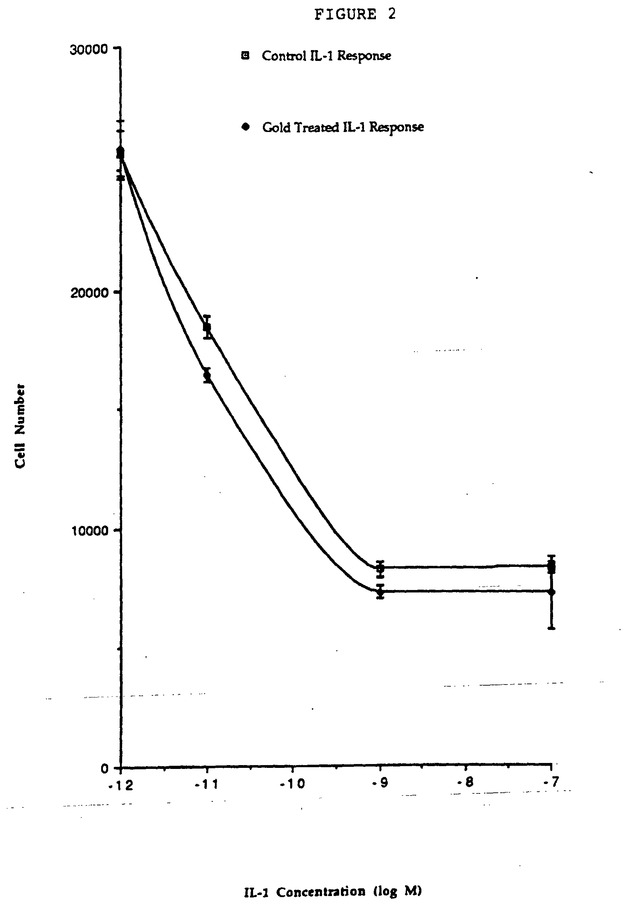

The following experiment shows the ability of IL-1 when mixed with colloidal gold to retain its biological activity by determining its ability to inhibit the growth of these cells. Approximately 8,000 MCF-7 cells were plated in 24-well tissue culture plates. On the next day, 15 nm gold particles were centrifuged at 14,000 rpms for 10 minutes and resuspended in sterile water. Human IL-1α was reconstituted in water to an initial stock of 5 X 10⁻⁵M in water. The pH of the gold and IL-1 was adjusted to approximately 8.0 with 0.1 M NaOH. Prior to mixing, the IL-1 was diluted to a working stock of 2 X 10⁻⁶, 2 X 10⁻⁸ M, and 2 X 10⁻¹⁰ M, which contained 250 µl of the gold (final volume = 0.5 ml). Gold controls consisted of 250 µl of gold and 250 µl of sterile water. Subsequently, each working stock was further diluted 1/20 in tissue media resulting in final concentrations of 10⁻⁷, 10⁻⁹, and 10⁻¹¹ M. These solutions along with the appropriate controls were then added directly to the MCF-7 cells. The data presented in Figure 2 are the number of cells present at various days after the addition of IL-1 with or without the gold.

### Example VII

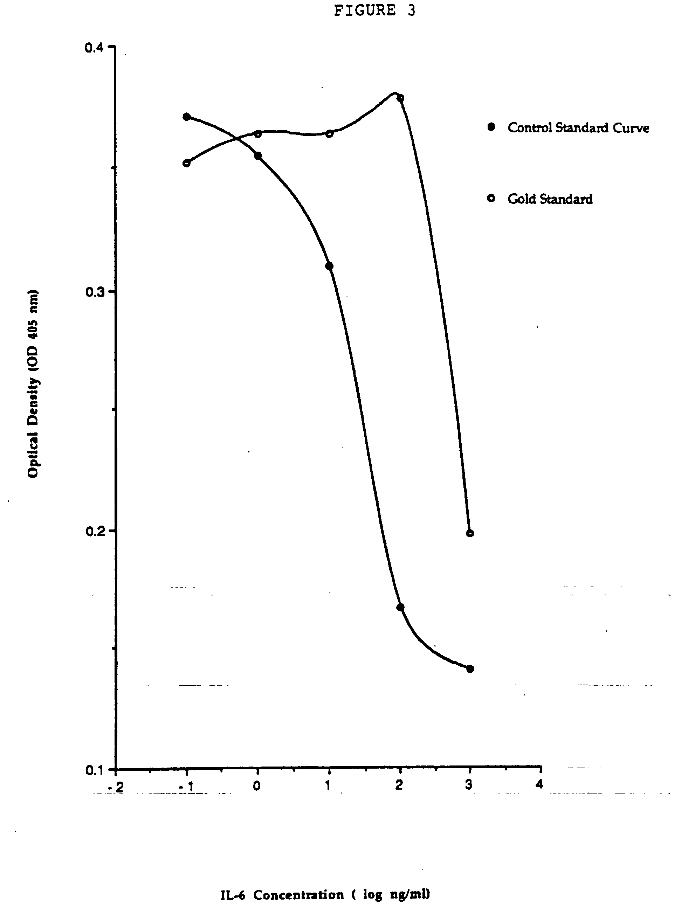

The following experiment shows the efficiency with which gold binds cytokine. This experiment demonstrates that the protein is removed from the solution when it is combined with gold and then centrifuged. The experiment used the IL-6 standard from ARI's Cytokit™₋₆. Prior to mixing, the pH of the gold and cytokine solutions were adjusted to pH 9 with 0.1 N NaOH. This protein was either preincubated with gold or water prior to using it in ARI's diagnostic kit for IL-6. Following this incubation, the colloidal gold/IL-6 mixture was centrifuged and the supernatants were used to generate a standard curve. As can be seen from Figure 3 the gold was very effective at binding virtually all the IL-6 in the dose-range of the assay, removing the IL-6 from the supernatant. Even at the highest final concentration (1000 ng/ml) of IL-6, the gold removed approximately 90% of the IL-6 in the solutions. This amount is based on the OD of the 1000 ng/ml IL-6/gold supernatant, which is similar to the 100 ng/ml IL-6 standard alone.

### Example VIII

The following experiment shows the physical changes in the gold colloid solution upon its mixing with TL-6, a potential antigen for a vaccine. Although the gold particles are approximately 15 nm in size, they cannot be filtered through a 0.22 µm syringe filter. We attribute this to the nature of the gold particles in this colloid mixture. It is theorized that the gold as a colloidal mixture forms aggregates larger than the individual spheres. Although the individual particles are smaller than the pore size of the filter, the aggregates are much larger and thus are not filterable. However, we observed that once the colloidal gold is incubated with protein it easily filters through the 0.22 µm filter. Thus, the binding of a cytokine appears to change the physical interactions of the gold particles with each other; making the gold particles act as single 15 nm particles and enabling the particles to be readily filtered. This experiment defines the nature of the binding of an antigen to the colloidal metal.

## Claims

1. A composition for therapeutic use comprising an admixture of a colloidal metal and an immunologically toxic biologically-active factor, selected from the group consisting of cytokines, growth factors, heat shock proteins, carbohydrate moieties of blood groups. Rh factors, glycoproteins from infectious organisms, inflammatory proteins, lipid A, endotoxins and immune regulatory proteins, wherein if the factor is selected from the group consisting of endotoxins, and wherein such endotoxin is lipopolysaccharide, the colloidal metal is not aluminium oxide.

2. The composition of claim 1, wherein the colloidal metal is selected from the group consisting of colloidal gold and colloidal silver.

3. The composition of claim 1 or 2 wherein the immunologically toxic biologically-active factor is selected from the group consisting of Interleukin-2 ("IL-2") phospholipase A2, staphylococcal entertoxin B, Type I Interferon, Type II Interferon. Tumor Necrosis Factor. IL-1, IL-6, IL-8, IL-4, Transforming Growth Factor-B, Lymphotoxin, IL-5, Migration Inhibition Factor, IL-3 Granolocyte-Macrophage Colony-Stimulating Factor ("CSF"), Monocyte-Macrophage CSF, Granulocyte CSF, IL-7, IL-10, IL-11, IL-12, IL-13, vascular epithelial growth factor ("VEGF"). Angiogenin, transforming growth factor ("TGFα"), heat shock proteins, carbohydrate moieties of blood groups, Rh factors, and fibroblast growth factor.

4. The composition of any of claims 1 to 3, further comprising a pharma-ceutically-acceptable component selected from the group consisting of excipients, buffers, antigen stabilizers, and sterilized carriers.

5. The composition of any of claims 1 to 4, further comprising a pharmaceutically-acceptable adjuvant.

6. The composition of any of claims 1 to 5, wherein the adjuvant is selected from the group consisting of Freund's Complete, lipopolysaccharide, monophophoryl lipid A, muramyl dipeptide, liposomes containing lipid A, alum, muramyl tripeptide-phosphatidyl-ethanoloamine, keyhole limpet hemocyanin, and Freund's Incomplete Adjuvant.

7. A vaccine comprising the composition of any of claims 1-6.

8. Use of any of the compositions of claims 1 to 6 for the preparation of a therapeutically active composition for the treatment of cancer, immune disease, viral disease or bacterial disease.

9. Use of colloidal metal to reduce toxic side effects of a biologically active factor in a therapeutically active composition comprising an immunologically toxic biologically active factor.

## Patentansprüche

1. Eine Zusammensetzung für eine therapeutische Verwendung, enthaltend eine Mischung eines kolloidalen Metalls und eines immunologisch toxischen, biologisch aktiven Faktors, ausgewählt aus der Gruppe, bestehend aus Cytokinen, Wachstumsfaktoren, Hitzeschockproteinen, Kohlenwasserstoffeinheiten von Blutgruppen, Rhesusfaktoren, Glycoproteinen von infektiösen Organismen, Entzündungsproteinen, Lipid A, Endotoxinen und Immun-Regulationsproteinen, wobei im Falle, dass der Faktor ausgewählt ist aus der Gruppe bestehend aus Endotoxinen und ein solches Endotoxin ein Lipopolysaccharid ist, das kolloidale Metall nicht Aluminiumoxid ist.

2. Die Zusammensetzung gemäß Anspruch 1, worin das kolloidale Metall ausgewählt ist aus der Gruppe bestehend aus kolloidalem Gold und kolloidalem Silber.

3. Eine Zusammensetzung gemäß Anspruch 1 oder 2, wobei der immunologisch toxische, biologisch aktive Faktor ausgewählt ist aus der Gruppe, bestehend aus Interleukin-2 ("IL-2"), Phospholipase A2, staphylokkischem Entertoxin B, Typ I Interferon, Typ II Interferon, Tumor-Nekrose-Faktor, IL-1, IL-6, IL-8, IL-4, transformierender Wachstumsfaktor-B, Lymphotoxin, IL-5, Migrationsinhibierungsfaktor, IL-3, Granulocyten-Macrophagen koloniestimulierender Faktor ("CSF"), Monocyten-Macrophagen-CSF, Granulocyten-CSF, IL-7, IL-10, IL-11, IL-12, IL-13, vaskulärer Epithelialwachstumsfaktor ("VEGF"), Angiogenin, transformierender Wachstumsfaktor ("TGFα"), Hitzeschockproteinen, Kohlenwasserstoffeinheiten von Blutgruppen, Rhesusfaktoren und Fibroblastenwachstumsfaktor.

4. Die Zusammensetzung gemäß einem der Ansprüche 1 bis 3, außerdem enthaltend eine pharmazeutisch annehmbare Komponente, ausgewählt aus der Gruppe, bestehend aus Arzneimittelträgern, Puffern, Antigen-Stabilisatoren und sterilisierten Trägern.

5. Die Zusammensetzung gemäß einem der Ansprüche 1 bis 4, außerdem enthaltend ein pharmazeutisch annehmbares Adjuvans.

6. Die Zusammensetzung gemäß einem der Ansprüche 1 bis 5, wobei das Adjuvans ausgewählt ist aus der Gruppe, bestehend aus Freund's komplettem, Lipopolysaccharid, Monophosphoryllipid A, Muramyldipeptid, Liposomen, enthaltend Lipid A, Alaun, Muramyltripeptid-Phosphatidyl-Ethanolamin, Schlüssellochnapfschnecken-Hämocyanin und Freund's inkomplettem Adjuvans.

7. Ein Impfstoff, enthaltend die Zusammensetzung gemäß einem der Ansprüche 1 bis 6.

8. Verwendung einer der Zusammensetzungen gemäß Anspruch 1 bis 6 für die Herstellung einer therapeutisch aktiven Zusammensetzung für die Behandlung von Krebs, einer Immunkrankheit, einer viralen Erkrankung oder einer bakteriellen Erkrankung.

9. Verwendung von kolloidalem Metall um die toxischen Nebenwirkungen eines biologisch aktiven Faktors in einer therapeutisch aktiven Zusammensetzung, die einen immunologisch toxischen, biologisch aktiven Faktor enthält, zu reduzieren.

## Revendications

1. Composition à usage thérapeutique, comprenant un mélange d'un métal colloïdal et d'un facteur biologiquement actif immunologiquement toxique, choisi dans le groupe constitué par les cytokines, facteurs de croissance, protéines de choc thermique, entités hydrate de carbone des groupes sanguins, facteurs Rh, glycoprotéines provenant d'organismes infectieux, protéines inflammatoires, lipide A, endotoxines et protéines régulatrices immunes, dans laquelle, si le facteur est choisi dans le groupe constitué par endotoxines et cet endotoxine est lipopolysaccharide, le métal colloïdal n'est pas d'oxyde d'aluminium.

2. Composition selon la revendication 1, dans laquelle le métal colloïdal est choisi dans le groupe constitué par l'or colloïdal et l'argent colloïdal.

3. Composition selon la revendication 1 ou 2, dans laquelle le facteur biologiquement actif immunologiquement toxique est choisi dans le groupe constitué par interleukine-2 ("IL-2"), phospholipase A2, enterotoxine staphylococcique B, Interféron type I, Interféron type II, facteur onconécrosant, IL-1, IL-6, IL-8, IL-4, facteur de croissance transformant B, lymphotoxine, IL-5, facteur d'inhibition de migration, IL-3, facteur de stimulation de colonie (« CSF ») de macrophage-granulocyte, CSF de macrophage-monocyte, CSF de granulocyte, IL-7, IL-10, IL-11, IL-12, IL-13, facteur de croissance épithéliale vasculaire ("VEGF"), angiogénine, facteur de croissance transformant ("TGF α"), protéines de choc thermique, entités hydrate de carbone des groupes sanguins, facteurs Rh, et facteur de croissance de fibroblaste.

4. Composition selon l'une quelconque des revendications 1 à 3, comprenant en outre un composant pharmaceutiquement acceptable choisi dans le groupe constitué par les excipients, tampons, stabilisateurs d'antigènes, et véhicules stérilisés.

5. Composition selon l'une quelconque des revendications 1 à 4, comprenant en outre un adjuvant pharmaceutiquement acceptable.

6. Composition selon l'une quelconque des revendications 1 à 5, dans laquelle l'adjuvant est choisi dans le groupe constitué par adjuvant complet de Freund, lipopolysaccharide, lipide A monophoryle, dipeptide muramyle, liposomes contenant le lipide A, de l'alun, un tripeptide muramyle éthanoloamine phosphatidyle, une hémocyanine KLH (« Keyhole Limpet Hemocyanin »), et adjuvant incomplet de Freund.

7. Vaccin comprenant la composition selon l'une quelconque des revendications 1-6.

8. Utilisation de l'une quelconque des compositions des revendications 1 à 6, pour la préparation d'une composition thérapeutiquement active pour le traitement d'un cancer, d'une affection immunologique, d'une maladie virale ou d'une maladie bactérienne.

9. Utilisation de métal colloïdal pour réduire les effets secondaires toxiques d'un facteur biologiquement actif dans une composition thérapeutiquement active comprenant un facteur biologiquement actif immunologiquement toxique.
